(19) Europäisches Patentamt
European Patent Office
Office européen des brevets

(11) **EP 2 301 462 B1**

(12) **EUROPEAN PATENT SPECIFICATION**

(45) Date of publication and mention
of the grant of the patent:
**05.07.2017 Bulletin 2017/27**

(51) Int Cl.:
***A61B 17/00*** (2006.01)   ***A61B 18/00*** (2006.01)
***A61B 18/12*** (2006.01)   ***A61B 18/14*** (2006.01)

(21) Application number: **10179353.7**

(22) Date of filing: **24.09.2010**

(54) **Electrosurgical generator for controlling output in response to voltage and current phases and for sustaining microbubble formation**

Elektrochirurgischer Generator zur Regelung der Ausgabe in Reaktion auf Spannungs- und Stromphasen und zur Aufrechterhaltung der Bildung von Mikroblasen

Générateur électrochirurgical pour contrôler une sortie en fonction des phases de la tension et du courant et pour maintenir la formation de microbulles

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **24.09.2009 US 566233**

(43) Date of publication of application:
**30.03.2011 Bulletin 2011/13**

(73) Proprietor: **Covidien LP**
**Mansfield, MA 02048 (US)**

(72) Inventor: **Gregg, William N.**
**Superior, CO 80027 (US)**

(74) Representative: **Soames, Candida Jane et al**
**Maschio & Soames IP Limited**
**30 Carlton Crescent**
**Southampton SO15 2EW (GB)**

(56) References cited:
**EP-A1- 1 707 144    US-A- 4 818 954**
**US-A- 5 423 808    US-A1- 2007 123 847**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

*1. Technical Field*

The present invention relates to an electrosurgical generator. More particularly, the present disclosure is directed to an electrosurgical generator for monitoring electrosurgical procedures based on real power.

*2. Background of Related Art*

Energy-based tissue treatment is well known in the art. Various types of energy (e.g., electrical, ohmic, resistive, ultrasonic, microwave, cryogenic, laser) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon that is applied to the tissue. A patient return electrode is placed remotely from the active electrode to carry the current back to the generator.

Ablation is most commonly a monopolar procedure that is particularly useful in the field of cancer treatment, where one or more RF ablation needle electrodes that (usually of elongated cylindrical geometry) are inserted into a living body. A typical form of such needle electrodes incorporates an insulated sheath disposed over an exposed (uninsulated) tip. When the RF energy is provided between the return electrode and the inserted ablation electrode, RF current flows from the needle electrode through the body. Typically, the current density is very high near the tip of the needle electrode, which tends to heat and destroy surrounding issue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact with body tissue with either of the separated electrodes prevents the flow of current.

Bipolar electrosurgical techniques and instruments can be used to coagulate blood vessels or tissue, e.g., soft tissue structures, such as lung, brain and intestine. A surgeon can either cauterize, coagulate/desiccate and/or simply reduce or slow bleeding, by controlling the intensity, frequency and duration of the electrosurgical energy applied between the electrodes and through the tissue. In order to achieve one of these desired surgical effects without causing unwanted charring of tissue at the surgical site or causing collateral damage to adjacent tissue, e.g., thermal spread, it is necessary to control the output from the electrosurgical generator, e.g., power, waveform, voltage, current, pulse rate.

It is known that measuring the electrical impedance and changes thereof across the tissue at the surgical site provides a good indication of the state of desiccation or drying of the tissue, e.g., as the tissue dries or loses moisture, the impedance across the tissue rises. This observation has been utilized in some electrosurgical generators to regulate the electrosurgical power based on measured tissue impedance.
The document US 5 423 808 A discloses an electrosurgical generator with a controller and with a power monitor.

## SUMMARY

An electrosurgical generator is provided for supplying electrosurgical energy to tissue, whereby the generator includes sensor circuitry configured to measure a voltage phase and a current phase through tissue and a processing device configured to compare the measured voltage and current phase to generate a real power component. The electrosurgical generator also includes a controller configured to regulate output of the electrosurgical generator based on the real power component and/or a predetermined imaginary impedance of tissue. Further features of the generator of the present invention are defined in claim 1.

## BRIEF DESCRIPTION OF THE DRAWINGS

Various embodiments of the present disclosure are described herein with reference to the drawings wherein:

Fig. 1A is a schematic block diagram of a monopolar electrosurgical system according to one embodiment of the present disclosure;
Fig. 1B is a schematic block diagram of a bipolar electrosurgical system according to one embodiment of the present disclosure;
Fig. 2 is a schematic block diagram of a generator according to an embodiment of the present disclosure; and
Fig. 3 is a flow chart of a method.

## DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

The generator according to the present disclosure can perform monopolar and bipolar electrosurgical procedures, including vessel sealing procedures. The generator may include a plurality of outputs for interfacing with various electrosurgical instruments (e.g., a monopolar active electrode, return electrode, bipolar electrosurgical forceps, footswitch). Further, the generator includes electronic circuitry configured for generating radio frequency power specifically suited for various electrosurgical modes (e.g., cutting, blending, division, ) and procedures (e.g., monopolar, bipolar, vessel sealing).

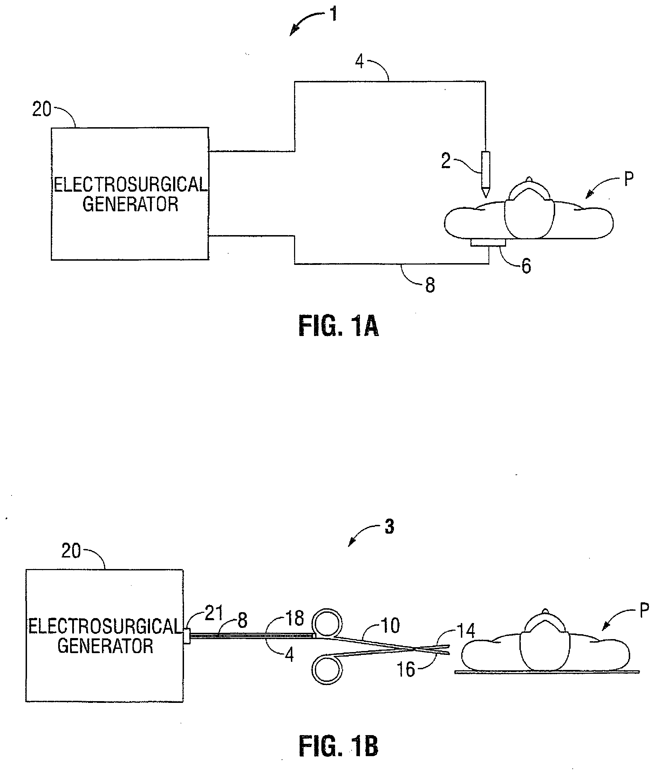

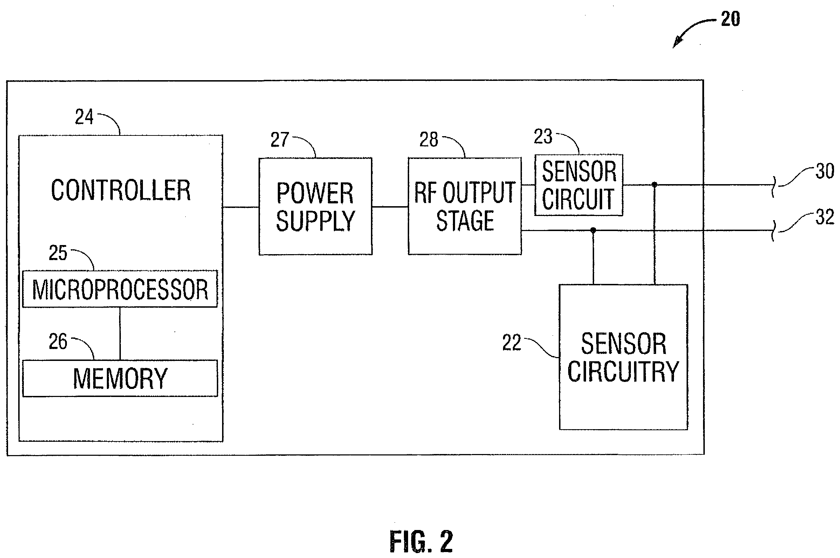

Fig. 1A is a schematic illustration of a monopolar electrosurgical system according to one embodiment of the present disclosure. The system includes an electrosurgical instrument 2 having one or more electrodes for treating tissue of a patient P. The instrument 2 is a monopolar type instrument including one or more active electrodes (e.g., electrosurgical cutting probe, ablation electrode(s)). Electrosurgical RF energy is supplied to the instrument 2 by a generator 20 via a supply line 4, which is connected to an active terminal 30 (Fig. 2) of the generator 20, allowing the instrument 2 to coagulate, seal, ablate and/or otherwise treat tissue. The energy is returned to the generator 20 through a return electrode 6 via a return line 8 at a return terminal 32 (Fig. 2) of the generator 20. The active terminal 30 and the return terminal 32 are connectors configured to interface with plugs (not explicitly shown) disposed at the ends of the supply line 4 and the return line 8, respectively.

The system may include a plurality of return electrodes 6 that are arranged to minimize the chances of tissue damage by maximizing the overall contact area with the patient P. In addition, the generator 20 and the return electrode 6 may be configured for monitoring 'tissue-to-patient' contact to insure that sufficient contact exists therebetween to further minimize chances of tissue damage.

Fig. 1B is a schematic illustration of a bipolar electrosurgical system according to the present disclosure. The system includes a bipolar electrosurgical forceps 10 having one or more electrodes for treating tissue of a patient P. The electrosurgical forceps 10 includes opposing jaw members having an active electrode 14 and a return electrode 16 disposed therein. The active electrode 14 and the return electrode 16 are connected to the generator 20 through cable 18, which includes the supply and return lines 4, 8 coupled to the active and return terminals 30, 32, respectively (Fig. 2). The electrosurgical forceps 10 is coupled to the generator 20 at a connector 21 having connections to the active and return terminals 30 and 32 (e.g., pins) via a plug disposed at the end of the cable 18, wherein the plug includes contacts from the supply and return lines 4, 8.

The generator 20 includes suitable input controls (e.g., buttons, activators, switches, touch screen) for controlling the generator 20. In addition, the generator 20 may include one or more display screens for providing the user with variety of output information (e.g., intensity settings, treatment complete indicators). The controls allow the user to adjust power of the RF energy, waveform parameters (e.g., crest factor, duty cycle), and other parameters to achieve the desired waveform suitable for a particular task (e.g., coagulating, tissue sealing, intensity setting). The instrument 2 may also include a plurality of input controls that may be redundant with certain input controls of the generator 20. Placing the input controls at the instrument 2 allows for easier and faster modification of RF energy parameters during the surgical procedure without requiring interaction with the generator 20.

Fig. 2 shows a schematic block diagram of the generator 20 having a controller 24, a high voltage DC power supply 27 ('HVPS') and an RF output stage 28. The HVPS 27 is connected to a conventional AC source (e.g., electrical wall outlet) and provides high voltage DC power to an RF output stage 28, which then converts high voltage DC power into RF energy and delivers the RF energy to the active terminal 30. The energy is returned thereto via the return terminal 32.

In particular, the RF output stage 28 generates waveforms (e.g., sinusoidal, square or any type of AC waveform) of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 typically generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The generator 20 may include a plurality of connectors to accommodate various types of electrosurgical instruments (e.g., instrument 2, electrosurgical forceps 10.). Further, the generator 20 is configured to operate in a variety of modes such as ablation, monopolar and bipolar cutting coagulation. The generator 20 may include a switching mechanism (e.g., relays) to switch the supply of RF energy between the connectors, such that, for instance, when the instrument 2 is connected to the generator 20, only the monopolar plug receives RF energy.

The controller 24 includes a microprocessor 25 operably connected to a memory 26, which may be volatile type memory (e.g., RAM) and/or non-volatile type memory (e.g., flash media, disk media). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed feedback control loop wherein sensor circuitry 22 and/or sensor circuitry 23, which may include a plurality of sensors for measuring a variety of tissue and energy properties (e.g., tissue impedance, tissue temperature, output current and/or voltage, voltage and current passing through the tissue, etc.), provides feedback to the controller 24. Such sensors are within the purview of those skilled in the art. The controller 24 signals the HVPS 27 and/or RF output stage 28, which then adjusts DC and/or RF power supply, respectively. The controller 24 also receives input signals from the input controls of the generator 20 or the instrument 2. The controller 24 utilizes the input signals to adjust power output by the generator 20 and/or performs other control functions thereon.

The present disclosure provides for a system for monitoring electrosurgical procedures using real power as opposed to reactive or imaginary power. The use of real power to control delivery of electrosurgical energy is discussed with respect to performing ablation procedures. Those skilled in the art will appreciate that the illustrated systems may be utilized with other electrosurgical procedures and/or modes.

Complex power consists of real and imaginary power components. Real power is identified with resistance or a purely resistive load and reactive or imaginary power is identified with reactance or a purely reactive load. Purely resistive impedance exhibits no phase shift between voltage and current, whereas reactance induces a phase shift θ between the voltage and the current passing through the tissue. More particularly, the phase shift θ is the angle by which the voltage phase leads the current phase.

Complex impedance consists of real and imaginary impedance. Real impedance is identified with resistance and imaginary impedance is identified with reactance. In addition, reactive impedance may be either inductive or capacitive. Purely resistive impedance exhibits no phase shift between the voltage and current, whereas reactance induces a phase shift θ between the voltage and the current passing through the tissue, thus imaginary impedance may be calculated based on the phase angle or phase shift between the voltage and current waveforms.

Electrosurgical generators sense various output parameters (e.g., voltage, current.) within the generator. As such, both cables connected to the generator and patient tissue are sensed by the generator as electrical loads. These electrical loads are typically complex loads due to loop inductance, leakage capacitance and complex tissue impedance. As a result, generator sensors do not accurately measure the tissue impedance but, rather, measure the total load associated with energy transmission. The amount of inaccuracy from this effect is dependent on the type of sensors employed and the differences between cable impedance and patient load impedance.

When attempting to control output power of an electrosurgical generator with the presence of the complex loads described above, as tissue resistance and total cable inductance change, the generator will adjust to the total impedance. This results in an inability of the generator to control tissue resistance and/or total cable inductance. A therapeutic effect requires that energy be delivered to the real component of the complex load since delivery of energy to the complex component results in stored energy in the reactive components rather than providing a direct therapeutic result. As a result, the energy delivered to the resistive component of the tissue will vary as the inductance of the cables vary, resulting in variable therapeutic outcomes.

The present disclosure provides for a method to address the above described variation in therapeutic effect by controlling the output of the generator based on the real component of the total load impedance and/or the real component of power delivered by the generator 20 to tissue. More specifically, the generator 20 obtains information (e.g., via a suitable sensor), such as complex impedance information for the cable and/or other suitable components, phase information related to the phase relationship between the current and voltage signals output by the generator 20 and information relating to the voltage and/or current output by the generator 20. The above stated information is used to calculate a real component of energy delivery that is, in turn, utilized to control generator 20 output.

The phase information describes a phase difference between current and voltage waveforms output by generator 20. The phase difference is determined by the microprocessor 25 by execution of a suitable software application, such as a single-band Fourier transform algorithm, a multi-band Fourier transform algorithm, an FFT algorithm, a Goertzel algorithm, an equivalent to a Fourier transform algorithm or a combination thereof. U.S. Patent No. 7,300,435 describes a control system that uses a Goertzel algorithm to determine the phase difference between the voltage waveform and the current waveform output by an electrosurgical generator. The phase difference is used to determine the real component of the total load impedance.

The generator 20 is configured to increase or decrease output to ensure that the current delivered to the resistive component of the total load impedance maintains the desired output power. In this manner, the effect of inductance changes in cables and/or other suitable components on energy delivered to the resistive component of tissue is minimized or substantially reduced. This results in a consistent therapeutic effect and less sensitivity to variations in cable routing and/or placement.

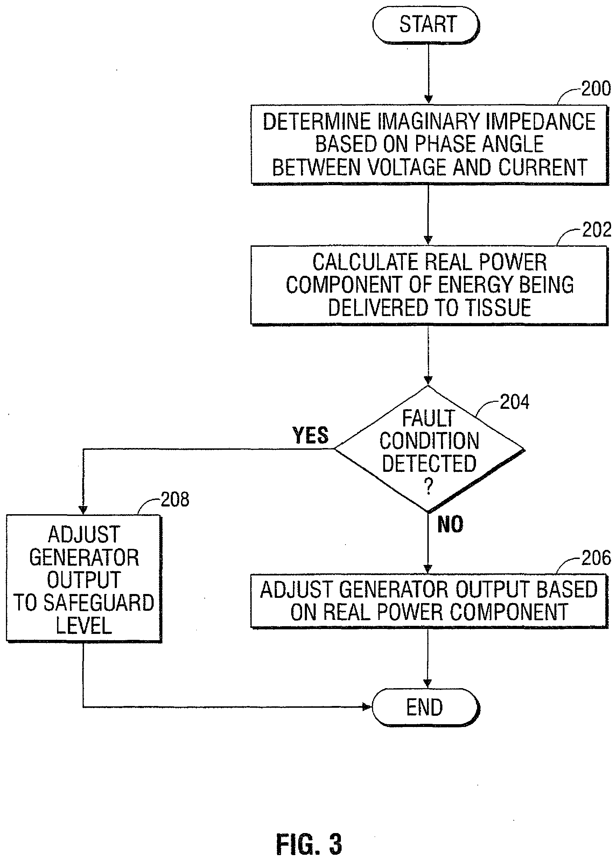

Fig. 3 shows a method for controlling output of the generator 20 based on the real power component of energy delivered by the generator 20 to tissue. The method may be implemessed as a software application embedded in the memory 26 and executed by the microprocessor 25 to control generator 20 output.

With reference to Fig. 3, in step 200, ablation energy is delivered to tissue and the imaginary impedance is measured by the sensor circuitry 22 and/or the sensor circuitry 23. More specifically, sensor circuitry 22 and/or sensor circuitry 23 measures voltage and current waveforms passing through the tissue and determines the imaginary impedance (e.g., the imaginary component of the complex impedance) based on the phase angle between the waveforms.

In step 202, the real power component of the ablation energy being delivered by the generator 20 is calculated using the following formula (1):

$$(1) \; P = VI \cos \theta$$

In formula (1), P is the real power component, V is the voltage phase passing through tissue, I is the current phase passing through tissue, and θ is the angle of phase shift θ between the voltage phase and the current phase passing through the tissue. If tissue is purely resistive (i.e., a purely resistive load), the phase shift between voltage phase and current phase is 0° and, since, cos θ = I, P=VI if tissue is purely resistive.

Typically, total power output of the generator 20 exceeds the desired power delivered to tissue. This excess or reactive power is stored as potential energy in the reactive components (e.g., cables) of the system. Formula (1) takes into account the real power component of the ablation energy being delivered by the generator 20, effectively disregarding the excess or reactive power. The calculated real power component is then processed by the microprocessor 25 as input data.

In step 204, specific parameters are monitored by the microprocessor 25 to detect a possible fault condition. In one case the fault condition may be a predetermined maximum range between complex impedance and real impedance (i.e., a maximum imaginary or reactive impedance). Changes in the imaginary impedance during energy delivery may be used as an indication of changes in tissue properties due to energy application. More specifically, imaginary impedance may be used to detect the formation of microbubbles, bubble fields and tissue desiccation that impart an electrical reactivity to the tissue that corresponds to sensed imaginary impedance. The tissue reactivity is reflective of the energy that is being delivered into the tissue. Thus, the measured change in imaginary impedance or the measured change between complex impedance and real impedance may be used as an indication of the amount of energy resident in the tissue. Monitoring of the resident energy in combination with monitoring of the energy being supplied by the generator allows for calculation of energy escaping the tissue during treatment, thereby allowing for determination of efficiency of the treatment process as well as any inadvertent energy drains.

According to the invention, output of the generator 20 can be controlled to a desired complex impedance to sustain microbubble formation. Changes in the complex impedance during energy delivery can be used as an indication of changes in tissue properties due to energy application. More specifically and as discussed above with reference to imaginary impedance, changes in complex impedance can also be used to detect the formation of microbubbles in that electrical reactivity imparted to tissue as a result of microbubble formation corresponds to changes in imaginary impedance. This change in imaginary impedance yields a corresponding change in complex impedance that may be monitored and used to control output of the generator 20.

According to the invention, the fault condition is a predetermined time limit that limits the amount of time for the generator 20 to achieve the desired power output level based on the real component of the total load impedance and/or the real component of power delivered by the generator 20 to tissue.

If the fault condition is detected in step 204, the fault condition is processed by the microprocessor 25 as input data and the controller 24 adjusts the output of the generator 20 to a pre-determined safeguard level in step 206. The safeguard level may be a discontinuance or termination of generator output or, alternatively, an output level pre-determined to be safe for the patient, the user, and/or components of the system should activation of generator 20 occur without a resistive load present or an inaccurate representation of real impedance be observed by the generator 20 due to a monitoring failure (e.g., sensor failure, microprocessor failure). Additionally or alternatively, generator output may be incrementally reduced in response to a detected fault condition such that for each incremental reduction in generator output, the microprocessor 25 re-checks for a possible fault condition. If the fault condition remains as detected by the microprocessor 25 following an increment of reduction, an additional increment of reduction is applied to generator output. If the fault condition is not detected by the microprocessor 25 following an increment of reduction, generator output is unchanged. In either case, the microprocessor 25 continues to monitor specific parameters for possible detection of a fault condition.

If the fault condition is not detected in step 204, in step 208 the real power component calculated in step 202 is processed by the microprocessor 25 as input data and the controller 24 adjusts the output of the generator 20 based on the processed real power component.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. The invention is defined by the claims appended hereto.

## Claims

1. An electrosurgical generator (20) for supplying electrosurgical energy to tissue, comprising:

   sensor circuitry (22) configured to measure a voltage phase and a current phase through tissue;
   a processing device (24) configured to compare the measured voltage and current phase to generate a real power component; and
   a controller (24) configured to regulate output of the electrosurgical generator based on the real power component and a predetermined imaginary impedance of tissue, whereby
   the controller is configured to adjust the output of the electrosurgical generator to a pre-determined safeguard level based on a detected fault condition, **characterized in that** the fault condition is the expiration of a predetermined time limit without the generator achieving a desired power output level based on the real power component, and **in that** the controller is configured to detect the formation of microbubbles in a given tissue based on changes in complex impendance and to subsequently control the output to ensure a desired complex impendance to sustain microbubble formation.

2. An electrosurgical generator according to claim 1, wherein the controller is configured to discontinue the output of the electrosurgical generator based on the predetermined imaginary impedance.

3. An electrosurgical generator according to claim 1 or 2, wherein the controller is configured to adjust the output of the electrosurgical generator to a predetermined level based on the predetermined imaginary impedance.

4. An electrosurgical generator according to any one of the preceding claims, wherein the processing device is configured to calculate the real power component based on a difference between the measured voltage phase and current phase.

5. An electrosurgical generator according to any one of the preceding claims, wherein the real power component corresponds to the output of the electrosurgical generator delivered to a resistive component of tissue.

6. An electrosurgical generator according to any one of the preceding claims, wherein the controller is configured to regulate output of the electrosurgical generator based on at least one of the real power component and a predetermined range between a complex impedance of tissue and a real impedance of tissue.

7. An electrosurgical generator according to claim 6, wherein the controller is configured to discontinue the output of the electrosurgical generator based on the predetermined range between the complex and real tissue impedance.

8. An electrosurgical generator according to claim 6 or 7, wherein the controller is configured to adjust the output of the electrosurgical generator to a predetermined level based on the predetermined range between the complex and real tissue impedance.

9. An electrosurgical generator according to claim 1, wherein the processing device is configured to calculate a difference between the voltage phase and the current phase and the controller is configured to calculate a real power component of the electrosurgical energy based on the difference between the voltage and current phases.

10. An electrosurgical generator according to claim 9, wherein the detected fault condition is the predetermined imaginary impedance of tissue.

11. An electrosurgical generator according to claim 9 or 10, wherein the controller is configured to discontinue the output of the electrosurgical generator based on the detected fault condition.

12. An electrosurgical generator according to claim 1, wherein the safeguard level is a discontinuance of generator output.

13. An electrosurgical generator according to claim 1, wherein the controller is configured so that generator output is incrementally reduced in response to the detected fault condition such that for each incremental reduction in generator output, the microprocessor re-checks for a possible fault condition, whereby if the fault condition remains as detected by the controller following an increment of reduction, an additional increment of reduction is applied to the generator output and if the fault condition is not detected by the controller following an increment of reduction, the generator output is unchanged.

## Patentansprüche

1. Elektrochirurgischer Generator (20) zur Zuführung von elektrochirurgischer Energie an Gewebe, umfassend:

   Sensorverschaltung (22), die konfiguriert ist, eine Spannungsphase und eine Stromphase durch Gewebe zu messen;

eine Bearbeitungsvorrichtung (24), die konfiguriert ist, die gemessene Spannungs- und Stromphase zu vergleichen, um eine reale Leistungskomponente zu erzeugen; und

einen Regler (24), der konfiguriert ist, den Output des elektrochirurgischen Generators auf der Basis der realen Leistungskomponente und einer vorgegebenen imaginären Impedanz von Gewebe zu regulieren, wodurch der Regler konfiguriert ist, den Output des elektrochirurgischen Generators auf ein vorgegebenes Schutzniveau auf der Basis eines festgestellten Fehlerzustands abzugleichen, dadurch charakterisiert, dass der Fehlerzustand der Ablauf einer vorgegebenen Frist ist, in welcher der Generator ein gewünschtes Leistungs-Outputniveau auf der Basis der realen Leistungskomponente nicht erreicht, und dadurch, dass der Regler konfiguriert ist, die Bildung von Mikroblasen in einem gegebenen Gewebe auf der Basis von Änderungen in der komplexen Impedanz festzustellen und anschließend den Output zu regeln, um eine gewünschte komplexe Impedanz zum Unterstützen der Mikroblasenbildung sicherzustellen.

2. Elektrochirurgischer Generator nach Anspruch 1, wobei der Regler konfiguriert ist, den Output des elektrochirurgischen Generators auf der Basis der vorgegebenen imaginären Impedanz abzustellen.

3. Elektrochirurgischer Generator nach Anspruch 1 oder 2, wobei der Regler konfiguriert ist, den Output des elektrochirurgischen Generators auf ein vorgegebenes Niveau auf der Basis der vorgegebenen imaginären Impedanz abzugleichen.

4. Elektrochirurgischer Generator nach einem der vorhergehenden Ansprüche, wobei die Verarbeitungsvorrichtung konfiguriert ist, die reale Leistungskomponente auf der Basis einer Differenz zwischen der gemessenen Spannungsphase und Stromphase zu errechnen.

5. Elektrochirurgischer Generator nach einem der vorhergehenden Ansprüche, wobei die reale Leistungskomponente dem Output des elektrochirurgischen Generators entspricht, der an eine resistive Komponente von Gewebe abgegeben wird.

6. Elektrochirurgischer Generator nach einem der vorhergehenden Ansprüche, wobei der Regler konfiguriert ist, den Output des elektrochirurgischen Generators auf der Basis von mindestens einem der realen Leistungskomponente und einer vorgegebenen Spanne zwischen einer komplexen Impedanz von Gewebe und einer realen Impedanz von Gewebe zu regulieren.

7. Elektrochirurgischer Generator nach Anspruch 6, wobei der Regler konfiguriert ist, den Output des elektrochirurgischen Generators auf der Basis der vorgegebenen Spanne zwischen der komplexen und realen Gewebeimpedanz einzustellen.

8. Elektrochirurgischer Generator nach Anspruch 6 oder 7, wobei der Regler konfiguriert ist, den Output des elektrochirurgischen Generators auf ein vorgegebenes Niveau auf der Basis der vorgegebenen Spanne zwischen der komplexen und realen Gewebeimpedanz abzugleichen.

9. Elektrochirurgischer Generator nach Anspruch 1, wobei die Verarbeitungsvorrichtung konfiguriert ist, eine Differenz zwischen der Spannungsphase und der Stromphase zu errechnen, und der Regler konfiguriert ist, die reale Leistungskomponente der elektrochirurgischen Energie auf der Basis der Differenz zwischen der Spannungs- und der Stromphase zu errechnen.

10. Elektrochirurgischer Generator nach Anspruch 9, wobei der festgestellte Fehlerzustand die vorgegebene imaginäre Impedanz von Gewebe ist.

11. Elektrochirurgischer Generator nach Anspruch 9 oder 10, wobei der Regler konfiguriert ist, den Output des elektrochirurgischen Generators auf der Basis des festgestellten Fehlerzustands einzustellen.

12. Elektrochirurgischer Generator nach Anspruch 1, wobei das Schutzniveau die Einstellung des Generator-Outputs ist.

13. Elektrochirurgischer Generator nach Anspruch 1, wobei der Regler so konfiguriert ist, dass der Generator-Output in Reaktion auf den festgestellten Fehlerzustand inkrementell reduziert wird derart, dass für jede inkrementelle Reduktion im Generator-Output der Mikroprozessor erneut auf einen möglichen Fehlerzustand prüft, wodurch, falls der Fehlerzustand wie vom Regler nach einem Reduktionsinkrement festgestellt verbleibt, ein zusätzliches Reduktionsinkrement am Generator-Output aufgebracht ist, und, falls der Fehlerzustand vom Regler nach einem Reduktionsinkrement nicht festgestellt wird, der Generator-Output unverändert ist.

**Revendications**

1. Générateur électrochirurgical (20) pour fournir de l'énergie électrochirurgicale à un tissu, comprenant :

un circuit de détection (22) configuré pour mesurer une phase de tension et une phase de courant à travers le tissu ;

un dispositif de traitement (24) configuré pour comparer la phase de tension et la phase de courant mesurées pour générer une composante de puissance réelle ; et

une unité de régulation (24) configurée pour réguler la sortie du générateur électrochirurgical sur la base de la composante de puissance réelle et d'une impédance imaginaire prédéterminée du tissu, de sorte que

le dispositif de commande soit configuré pour ajuster la sortie du générateur électrochirurgical à un niveau de sauvegarde prédéterminé sur la base d'un état de défaillance détecté, **caractérisé en ce que** l'état de défaillance est l'expiration d'une limite de temps prédéterminée sans que le générateur n'atteigne un niveau de sortie de puissance souhaité sur la base de la composante de puissance réelle et **en ce que** l'unité de régulation est configurée pour détecter la formation de microbulles dans un tissu donné sur la base de changements d'impédance complexe et réguler ensuite la sortie pour assurer une impédance complexe souhaitée pour soutenir la formation de microbulles.

2. Générateur électrochirurgical selon la revendication 1, dans lequel l'unité de régulation est configurée pour interrompre la sortie du générateur électrochirurgical sur la base de l'impédance imaginaire prédéterminée.

3. Générateur électrochirurgical selon la revendication 1 ou 2, dans lequel l'unité de régulation est configurée pour ajuster la sortie du générateur électrochirurgical à un niveau prédéterminé sur la base de l'impédance imaginaire prédéterminée.

4. Générateur électrochirurgical selon l'une quelconque des revendications précédentes, dans lequel le dispositif de traitement est configuré pour calculer la composante de puissance réelle sur la base d'une différence entre la phase de tension et la phase de courant mesurées.

5. Générateur électrochirurgical selon l'une quelconque des revendications précédentes, dans lequel la composante de puissance réelle correspond à la sortie du générateur électrochirurgical délivrée à un composant résistif de tissu.

6. Générateur électrochirurgical selon l'une quelconque des revendications précédentes, dans lequel l'unité de régulation est configurée pour réguler la sortie du générateur électrochirurgical sur la base d'au moins l'une parmi la composante de puissance réelle et une plage prédéterminée entre une impédance complexe du tissu et une impédance réelle du tissu.

7. Générateur électrochirurgical selon la revendication 6, dans lequel l'unité de régulation est configurée pour

interrompre la sortie du générateur électrochirurgical sur la base de la plage prédéterminée entre l'impédance complexe du tissu et l'impédance réelle du tissu.

8. Générateur électrochirurgical selon la revendication 6 ou 7, dans lequel l'unité de régulation est configurée pour

ajuster la sortie du générateur électrochirurgical à un niveau prédéterminé sur la base de la plage prédéterminée entre l'impédance complexe et l'impédance réelle du tissu.

9. Générateur électrochirurgical selon la revendication 1,

dans lequel le dispositif de traitement est configuré pour calculer une différence entre la phase de tension et la phase de courant et

l'unité de régulation est configurée pour calculer une composante de puissance réelle de l'énergie électrochirurgicale sur la base de la différence entre les phases de tension et de courant.

10. Générateur électrochirurgical selon la revendication 9, dans lequel l'état de défaillance détecté est l'impédance imaginaire prédéterminée du tissu.

11. Générateur électrochirurgical selon la revendication 9 ou 10, dans lequel l'unité de régulation est configurée pour interrompre la sortie du générateur électrochirurgical sur la base de l'état de défaillance détecté.

12. Générateur électrochirurgical selon la revendication 1, dans lequel le niveau de sauvegarde est une interruption de la sortie du générateur.

13. Générateur électrochirurgical selon la revendication 1, dans lequel l'unité de régulation est configurée de sorte que la sortie du générateur soit réduite par incréments en réponse à l'état de défaillance détecté de sorte que, pour chaque réduction incrémentale de la sortie du générateur, le microprocesseur revérifie un état de défaillance possible, en sorte que, si l'état de défaillance subsiste comme détecté par l'unité de régulation après un incrément de réduction, un incrément de réduction supplémentaire soit appliqué à la sortie du générateur et que, si l'état de défaillance n'est pas détecté par l'unité de régulation à la suite d'un incrément de réduction, la sortie du générateur reste inchangée.

**FIG. 1A**

**FIG. 1B**

**FIG. 2**

EP 2 301 462 B1

START

DETERMINE IMAGINARY IMPEDANCE
BASED ON PHASE ANGLE
BETWEEN VOLTAGE AND CURRENT
─200

CALCULATE REAL POWER
COMPONENT OF ENERGY BEING
DELIVERED TO TISSUE
─202

FAULT
CONDITION
DETECTED
?
─204

YES

NO

ADJUST
GENERATOR
OUTPUT
TO SAFEGUARD
LEVEL
─208

ADJUST GENERATOR OUTPUT BASED
ON REAL POWER COMPONENT
─206

END

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5423808 A

- US 7300435 B